# EUROPEAN PATENT SPECIFICATION

(11) **EP 1 609 488 B1**
(45) Date of publication and mention of the grant of the patent: **28.05.2008**
(21) Application number: 03715678.3
(22) Date of filing: 31.03.2003
(51) Int. Cl.: A61L 2/28, A61L 2/14, G01N 31/22

(54) **PACKAGING MATERIAL WITH INDICATOR FOR HYDROGEN PEROXIDE STERILIZATION**
VERPACKUNGSMATERIAL MIT INDIKATOR FÜR WASSERSTOFFPEROXID-PLASMASTERILISATION
MATERIAU D'EMBALLAGE AVEC UN INDICATEUR POUR LA STERILISATION DE PLASMA DE PEROXYDE D'HYDROGENE

(43) Date of publication of application: 28.12.2005
(73) Proprietor: FUJIMORI KOGYO CO., LTD., Tokyo 103-0002 (JP)
(72) Inventor: SUTOH, Teiko c/o Fujimori Kogyo Co., Ltd., Chuo-ku, Tokyo 103-0002 (JP); SUGIYAMA, Hiroko c/o Fujimori Kogyo Co., Ltd., Chuo-ku, Tokyo 103-0002 (JP); HAYASHI, Masushi c/o Fujimori Kogyo Co., Ltd., Chuo-ku, Tokyo 103-0002 (JP)
(74) Representative: Fuhlendorf, Jörn
(86) International application number: PCT/JP2003/004129
(87) International publication number: WO 2004/087222

(56) References cited:
- WO-A-01/10471
- WO-A-01/86289
- WO-A1-00/61200
- WO-A1-01/40792
- JP-A- 2002 303 618
- US-A1- 2002 051 733
- US-B1- 6 428 748

## Description

### FIELD OF THE INVENTION

The present invention relates to a sterilization indicator using a chemical indicator that is useful in hydrogen peroxide-plasma sterilization treatment and a sterilization packaging material for storing an article to be sterilized by plasma sterilization treatment that has an indicator area formed from the sterilization indicator.

### BACKGROUND OF THE INVENTION

Various sterilization means have been used for sterilization of, for example, disposable or reusable medical devices and food containers, and typical examples thereof include ethylene oxide gas (EOG) sterilization, high-pressure high-temperature steam sterilization in an autoclave, plasma sterilization, and the like. Typically as the sterilization method practiced in hospitals and the like, articles to be sterilized such as knives and syringes are contained and sealed in a packaging bag having an area that allows permeation of at least gases but no microbes and sterilized, for example, by one of the methods described above, and the articles after sterilization are kept in the packaging bag until used, for example, in a surgical operation and are taken out from the bag before use in an operation or treatment. In such a case, it is useful to place on the packaging material a display region allowing easy recognition of whether or not the article sealed therein is already sterilized, from the viewpoint of prevention of accidents. The indicator area is formed by using an ink having an indicator function that is discolored irreversibly by sterilization treatment.

The following documents are related to the subject-matter of the invention:
Document WO0186289 discloses an indicator suitable for monitoring oxidizing vapour or plasma for medical devices, the indicator contains polymers comprising activator which causes an indicator to change colour on reaction with the oxidizing plasma.
Document US6428748 discloses a detector for monitoring an analyte which includes an analyte-sensing composition. The analyte-sensing composition has a visible colour intensity or emission intensity (e.g., fluorescence intensity) that changes as the analyte concentration contacting the detector changes. The intensity changes can be visible to the human eye, or identified by an instrument.
Document WO0110471 discloses a device for monitoring sterilization with ethylene oxide comprising at least one layer of polymer, having incorporated therein an indicator like bromothymol blue, bromocresol purple, capable of undergoing at least one color change, an activator for the indicator consisting of a halide like sodium bromide, tetrabutylammonium bromide. In case the activator is contacted with ethylene oxide, it undergoes a reaction with it to produce a product which causes the indicator to undergo a colour change.

Examples of the temperature-sensitive colorants used in such inks as indicators of steam sterilization include colorants for thermal-recording materials such as diazonium salt compounds and colorants encapsulated in a thermosensitive microcapsule that develops colors when heated to a designated temperature as the microcapsular sealing is broken, and the like; and examples of such products commercially available include Nescos IC (S-25B, for high-pressure vapor sterilization) manufactured by I.C.S center (Sakura Color Products Corp.). And inks used for thermo-sensitive labels and steam integrator cards manufactured by Nichiyu Giken Kogyo Co., Ltd., and the like. The EOG-sensitive inks include, for example, the compositions of dispersion dyes having the (A-N=N-B) structure described in Japanese Patent Application Laid-Open (JP-A) No. 5-1252.

A hydrogen peroxide plasma sterilization method and an apparatus therefor have recently been proposed and actually used (Japanese Patent Application Publication (JP-B) Nos. 2-62261 and 7-22693) because, among the above sterilization treatments, the method raises fewer concerns about adverse effects on the articles to be sterilized, i.e., medical devices. In brief, in the sterilization method, an article to be sterilized in an airtight container is brought into contact with hydrogen peroxide vapor under reduced pressure and hydrogen peroxide plasma is generated. This method is very useful because it gives high sterilization efficiency and, moreover, hydrogen peroxide is converted completely to harmless water and oxygen.

Examples of the indicator compositions which have been proposed that are compatible withsuitable to the plasma sterilization treatment include the triphenylmethane or the cyanine colorant described in JP-A No. 11-178904, the discoloration colorant described in JP-A No. 11-37988 as a pH indicator, and the like.

These indicator compositions, which include a colorant that is decolored or discolored by the oxidative force of a highly oxidative gas used in the plasma sterilization, have had problems such as lower discoloration speed, insufficient discoloration by short-term plasma sterilization treatment, insufficient light stability, and undesirable discoloration by handling under white light or daylight, and thus have not been favorable for practical use.

For that reason, using of a pH indicator that changes its color according to pH was studied as an indicator higher in discoloration speed, but a sterilization bag having such an indicator area has a problem of lack of stability, because the discoloration reaction is reversible and the color of the indicator area changes according to the storage environment, resulting in difficulty in determining after sterilization whether the article therein is actually sterilized or not.

An object of the present invention, which was devised in consideration of the traditional problems, is to provide a plasma-sterilization indicator allowing definite judgment of whether or not an article to be sterilized has been processed with plasma sterilization treatment that is superior in stability including light stability, that has significant color change and is irreversible, and is higher in indicator discoloration speed, and a sterilization packaging material having an indicator area using the plasma-sterilization indicator.

### SUMMARY OF THE INVENTION

The sterilization packaging material according to the present invention is capable of containing an article to be sterilized by plasma-sterilization treatment, wherein at least part thereof is made of a gas permeable paper or nonwoven fabric, and an indicator area of a hydrogen peroxide plasma-sterilization indicator comprising one or more compounds (A) selected from the group consisting of absorption indicators and metal chelate tritration indicators wherein the one or more compounds (A) is selected from hematoxylin, Mordant Blue 29, Eriochrome Black T, xylenol orange, and 1-(2-pyridylazo)-2-naphthol (PAN), and an organic metal compound (B) which is one or more compound selected from aluminium chelate compounds, titanium chelate compounds, and zirconium chelate compounds is formed thereon.

The indicator area may further comprise polyvalent alcohol (C).

After studies, the inventors have found that it was possible to solve the problems aforementioned by using a particular organic metal compound and completed the present invention. Namely, the plasma-sterilization indicator according to the invention comprising: one or more compounds (A) selected from the group consisting of adsorption indicators and metal chelate-titration indicators, and an organic metal compound (B).

In another embodiment, the plasma-sterilization indicator according to the invention comprising: one or more compounds (A) selected from the group consisting of adsorption indicators and metal chelate-titration indicators, an organic metal compound (B), and a polyvalent alcohol (C).

The one or more compounds (A) selected from the group consisting of adsorption indicators and metal chelate-titration indicators for use as the plasma-sterilization indicator are preferably compounds selected from hematoxylin, Mordant Blue 29, Eriochrome Black T, xylenol orange, and 1-(2-pyridylazo)-2 naphthol (PAN).

### BEST MODE OF CARRYING OUT THE INVENTION

The first embodiment of the plasma-sterilization indicator according to the present invention, i.e., the plasma-sterilization indicator including one or more compounds (A) selected from the group consisting of adsorption indicators and metal chelate-titration indicators and an organic metal compound (B), will be described first.

The plasma-sterilization indicator according to the invention, which uses adsorption indicators and a metal chelate-titration indicator as discoloration compounds, is almost free from the concern about discoloration under normal white light or daylight; the product formed in the reaction between the indicator and a co-present organic metal compound discolors into a definitely different color in a particular pH range by the pH change caused by hydrogen peroxide and the oxidative force in plasma treatment, and the discoloration is irreversible; and thus the plasma-sterilization indicator is favorable as a chemical indicator and allows definite judgment of whether an article to be sterilized was sterilized even after a certain period from hydrogen peroxide plasma sterilization treatment of the article, such as medical devices and food containers, to be sterilized.

The one or more compounds (A) selected from the group consisting of adsorption indicators and metal chelate-titration indicators for use are preferably compounds selected from hematoxylin, Mordant Blue 29, Eriochrome Black T, xylenol orange, and 1-(2-pyridylazo)-2 naphthol (PAN).

The one or more compounds (A) selected from the group consisting of adsorption indicators and metal chelate-titration indicators will be described below in detail.

The adsorption indicator (A-1) for use in the invention is not particularly limited, and any one of common indicators used in detection of metal ions and others that discolor as adsorbed on colloidal particle may be used favorably. Examples of the adsorption indicator for use in the invention include congo red, phenol red, methyl red, bromocresol purple, bromophenol blue, titanium yellow, eosin, fluorescein, dichlorofluorescein, dibromofluorescein, aluminon, alizarin, curcumin, and the like.

The metal chelate-titration indicator (A-2) for use in the invention is selected from organic colorants that discolor by binding to a metal ion forming a complex ion (colorant compounds having a proton that can be replaced with a metal ion in the molecule) and compounds having multidentate ligands that can form a chelate compound by binding to a metal ion. The chelate-titration indicator may be any one of polyaminocarboxylic acids, oxycarboxylic acids, water-soluble compounds represented by condensed phosphate salts, and compounds scarcely-soluble in water represented by dimethylglyoxime, oxine, dithizone, and the like.

Specific examples of the metal chelate-titration indicators for use in the invention include Tiron, Mordant Blue 29, Eriochrome Black T, xylenol orange, Alizarin red S, N-benzoyl-N-phenylhydroxylamine, 5-sulfosalicylic acid dihydrate, sulfosalicylic acid dihydrate, 1-(2-pyridylazo)-2-naphthol (hereinafter, referred to as PAN), murexide, hematoxylin, disodium dihydrogen ethylenediaminetetraacetate dihydrate (EDTA), phthalein complexones, dimethylglyoxime, oxine, dithizone, methyl thymol blue, and the like.

Among them, the compounds preferable in the invention are hematoxylin, Mordant Blue 29, Eriochrome Black T, xylenol orange, PAN, and the like; and among them, hematoxylin is more preferable from the viewpoints of availability and easiness in detecting the color change between before and after sterilization.

These compounds may be used alone or in combination of two or more as needed.

In the plasma-sterilization indicator according to the invention, the content of the one or more compounds (A) selected from the group consisting of adsorption indicators and metal chelate-titration indicators is decided according to the relationship with the organic metal compound (B) described in detail below, but generally, approximately in the range of 0.2 to 10.0 wt % and more preferably in the range of 0.5 to 5.0 wt % as solid matter.

A content of less than 0.2 wt %, which may lead to deterioration in light stability and also in density of the color and visibility of the indicator area after the discoloration due to sterilization, and a content of more than 10.0 wt %, which may lead to generation of undissolved matters when a coating solution for forming the indicator area is prepared, are both unfavorable.

The organic metal compound (B) will be described next.

The organic metal compound (B) favorably used in the invention is a compound that discolors definitely in reaction with the one or more compounds (A) selected from the group consisting of adsorption indicators and metal chelate-titration indicators in the presence of acid; preferable examples thereof include compounds selected from the group consisting of aluminum chelate compounds, titanium chelate compounds, and zirconium chelate compounds; and typical examples thereof include aluminum chelate compounds such as aluminum ethyl acetoacetate, titanium chelate compounds such as diisopropoxybis(acetylacetonato)titanium, and tris(acetylacetonato)titanium, zirconium chelate compounds such as acetylacetone tributoxyzirconium, and the like.

Typical examples of the organic metal compounds favorably used in the invention include aluminum chelate compounds such as aluminum ethyl acetoacetate, aluminum tris(ethylacetoacetate), aluminum tris(acetylacetonate), and aluminum bisethylacetoacetate monoacetylacetonate; titanium chelate compounds such as diisopropoxybis(acetylacetonato)titanium, isopropoxy(2-ethyl-1,3-hexanediolato)titanium, diisopropoxybis(triethanolaluminato)titanium, di(2-ethylhexoxy)bis(2-ethyl-1,3-hexanediolato)titanium, di-N-butoxybis(triethanolaluminato)titanium, and titanium tetraacetylacetonate; acetylacetone tributoxyzirconium; and the like. Preferable are compounds selected from aluminum and titanium chelate compounds having a chelating ability; and among them, aluminum ethyl acetoacetate diisopropylate and diisopropoxybis(acetylacetonato)titanium are particularly preferable.

These compounds may be used alone or in combination of two or more.

The content of these compounds is generally, approximately in the range of 0.2 to 5.0 wt %, preferably 0.5 to 3.0 wt %, as solid matter. A content of less than 0.2 wt %, which may make the color before plasma sterilization unstable, and a content of more than 5.0 wt %, which may lead, for example, to change in the color before plasma sterilization over time or increase in the viscosity of the indicator ink after preparation, causing problems in ink stability, are both unfavorable.

The plasma-sterilization indicator according to the invention including both the components (A) and (B) discolors irreversibly by pH change or application of a strong oxidative force, and thus, has properties favorable as an indicator.

As for typical discoloration, for example when phenol red is used as the component (A) and aluminum ethyl acetoacetate diisopropylate as the component (B), the color before sterilization is yellowish red, while the color after sterilization is changed to red. Alternatively, when the same compound is used as the component (B), the color changes from gray to yellow if Eriochrome Black T is used as the component (A) and from dark brown to yellow if hematoxylin is used as the component (A). Both of these changes allow visual detection of discoloration.

Hereinafter, the second embodiment of the plasma-sterilization indicator according to the invention, i.e., the plasma-sterilization indicator including one or more compounds (A) selected from the group consisting of adsorption indicators and metal chelate-titration indicators, an organic metal compound (B), and a polyvalent alcohol (C), will be described.

The one or more compounds (A) selected from the group consisting of adsorption indicators and metal chelate-titiation indicators and the organic metal compound (B) in the second embodiment are the same as those used in the first embodiment, and the preferable contents are also the same.

The polyvalent alcohols (C) for use in the second embodiment include, for example, one or more glycols selected from ethylene glycol, diethylene glycol, polyethylene glycol, propylene glycol, and dipropylene glycol.

It is possible to control the discoloration speed of the indicator according to the invention by addition of these compounds. These compounds are effective in increasing the discoloration speed, and among them, polyethylene glycol, diethylene glycol, and the like are superior in increasing the discoloration speed, while ethylene glycol, propylene glycol, and the like are moderate in increasing the discoloration speed.

These polyvalent alcohols may be used alone or in combination of two or more if they are compatible with each other.

It is possible to increase the speed desirably by adjusting the kinds and amounts of these compounds.

The content of these compounds is generally, approximately in the range of 1.0 to 10.0 wt % and preferably 2.0 to 5.0 wt %. An excessively smaller content, which may result in insufficient increase in the discoloration speed during plasma sterilization, and a content of more than 10.0 wt %, which is not effective any more and rather causes problems in ink stability, for example, by leading to decrease in the viscosity of the indicator ink, are both undesirable.

The plasma-sterilization indicator including the compounds (A), (B) and (C) in the second embodiment of the invention discolors irreversibly and rapidly by pH change and application of a strong oxidative force and thus, has properties favorable as an indicator.

In either the first or second embodiment, the sterilization indicator according to the invention may include another compound commonly used as a binder, in addition to the essential components above.

The sterilization indicator according to the invention preferably includes a binder. The binder represents a carrier when an indicator is used as an indicator area of packaging material.

The binder is not particularly limited and is used as selected, for example, from synthetic resins commonly used in preparation of printing ink.

The indicator according to the invention is preferably an alkali-resistant polymer material such as a polyamide or ethylcellulose binder from the viewpoint of the stability as a composition, but is not necessarily alkali resistant because it is possible to prevent the damage due to alkaline components by adding other additives during preparation. Thus, considering the discoloration speed and the stability of colorant, nitrocotton, for example, may be used favorably as the binder.

The kind and the addition amount of the binder may be selected properly according, for example, to the desirable properties as a sterilization indicator, the material for the packaging material or container to be applied, and the desirable durability thereof, but the addition amount is generally, approximately 5 to 30 wt %.

In addition to the respective components, the plasma-sterilization indicator according to the invention may further include as needed additives such as known plasticizer, dispersant, stabilizer, and thickener, for improvement in durability and the convenience in handling.

In particular, the plasma-sterilization indicator according to the invention preferably includes an ultraviolet absorbent, from the viewpoint of improvement in light stability. Any one of ultraviolet absorbents commonly used in the art may be used as the ultraviolet absorbent, if it does not show adverse effects on achieving the object of the invention. Typical favorable examples thereof include triazine compounds, benztriazole compounds, hindered amine compounds, and the like. Commercially available products of such compounds having an ultraviolet ray-blocking effect include various compounds known in the trade name of Tinuvin manufactured by Ciba-Geigy Corp.

The ultraviolet absorbents may be used alone or in combination of two or more, but it is also effective to use multiple ultraviolet absorbents having different properties.

The plasma-sterilization indicator according to the invention is a composition that discolors by plasma sterilization treatment, but, if the color of the indicator after discoloration is weak, a dye or pigment that does not affect the color of the principal colorant component and does not decolor or discolor by sterilization treatment may be added for improvement of visibility. For example, it is preferable to add a yellow dye or pigment in a suitable amount when the color after discoloration is yellow or light yellow.

The plasma-sterilization indicator according to the invention can be used as coated and dried on the area where the indicator is needed after the respective components are dissolved in a favorable solvent.

The solvent is selected considering the solubility of the anthraquinone colorant and the other components described above, but ethanol, methanol, ethyl acetate, isopropanol, n-propanol, toluene, and the like are preferable from the points of the stability of colorant and the efficiency in drying during printing.

The sterilization packaging material according to the invention will be described next. The sterilization packaging material according to the invention is a sterilization packaging material capable of containing an article to be sterilized by plasma sterilization treatment and at least part thereof being made of a gas permeable paper or nonwoven fabric, characterized by having an indicator area formed thereon of a plasma-sterilization indicator including one or more compounds (A) selected from the group consisting of adsorption indicators and metal chelate-titration indicators, an organic metal compound (B), and as needed a polyvalent alcohol (C).

The packaging material can be prepared by selecting arbitrarily a known sheet having a sterilizable area, at least a part of which is made of a gas permeable base material, and a desirable strength. Examples of the gas permeable base materials include paper, porous film, woven fabric, nonwoven fabric and the like, and among them, paper or nonwoven fabric are preferable. However, the gas permeable base material is preferably made of a raw material that does not adsorbs H₂O₂ used in sterilization treatment.

The packaging material should have a sterilizable area, that is, an area of a paper or nonwoven fabric having a permeability that allows permeation of an effective ingredient gas but of no microbe during the plasma sterilization treatment applied in the present invention.

Although the entire packaging material may be prepared from such a sterilizable material, use of a gas permeability material such as paper or nonwoven fabric in one side and a transparent synthetic resin sheet in the other side is also a preferred embodiment, from the viewpoints of cost, strength, and the visibility of content. For example, a bag-shaped gas permeable base material having two kinds of sheets different in function, a gas permeable paper or nonwoven fabric sheet in one side and a transparent synthetic resin sheet on the other, has advantages in easiness of production, superiority in the visibility of content, and the secured strength of the packaging material produced.

The sterilization packaging material according to the invention is not particularly limited if at least a part thereof is made of a gas permeable base material, but as described above, it is preferable to laminate two sheets for front and rear faces and to form peelable adhesion areas close to both ends and an indicator area of the indicator described above on at least one sheet, from the viewpoints of the convenience in productivity and in handling of the packaging material obtained. The packaging material after the processing above is converted into and supplied in any desirable shape as needed, for example, of bags or rolls.

The indicator area on the packaging material is formed normally by coating and drying at least one plasma-sterilization indicator composition dissolved in a suitable solvent on a sheet of packaging material.

The indicator area thus formed may have additionally a surface protective layer for protection of the indicator area. The surface protective layer may be formed with any material if it is a transparent or semi-transparent material that permeates the hydrogen peroxide vapor used in plasma sterilization treatment or the hydrogen peroxide-derived plasma and allows recognition of the change in colorant color.

In particular, it is preferably formed with a composition including a binder for the composition forming an indicator area as a film-forming component and additionally an ultraviolet absorbent, a water-repellent component for water tightness, and an abrasion-resistant component, specifically a wax component such as polyethylene wax, from the viewpoint of the compatibility with the indicator area. The surface protective layer on indicator area may also be formed by coating a solution thereof in a similar manner to the formation of the indicator area.

The indicator area on sterilization packaging material is normally formed on the surface of the gas permeable paper or the non-woven fabric sheet occupying part of the packaging material. When the other constituent material of the packaging material is a transparent sheet allowing observation inside through the material, the layer of the indicator area may be formed inside of the gas permeable paper or the non-woven fabric sheet or the transparent resin sheet (i.e., inside of the bag formed with the packaging material).

The composition above for the indicator is blended uniformly by any one of known kneading methods practiced in the art for production of printing inks, and the mixture is then coated on a sheet for packaging material, forming the indicator area. Examples of the application methods include known printing methods, for example, offset printing, flexographic printing, gravure printing, and the like. The surface protective layer on the indicator area may also be formed in a similar manner.

The amount of the coating solution for the indicator area is not particularly limited and may be selected arbitrarily, as far as the indicator area is visually recognizable, but generally, approximately in the range of 0.2 to 20 g/m² and preferably 1 to 10 g/m². An excessively low coating amount may result in deterioration in visibility after sterilization treatment, while an excessively high coating amount in deterioration in the scuff resistance during transportation or storage.

Alternatively, the amount of the coating solution for the surface protective layer formed as needed is preferably in the range of 1 to 10 g/m².

### EXAMPLES

Hereinafter, the present invention will be described in detail with reference to typical Examples, but it should be understood that the invention is not restricted thereby. In the following Examples, "%" means "wt %", unless specified otherwise.

### (Example 1)

The following components for a plasma-sterilization indicator were mixed until homogeneity, to give a composition for forming an indicator area.
(Plasma-sterilization indicator composition 1)
   - Hematoxylin 1.0 wt %
   - Diisopropoxybis(acetylacetonato)titanium 1.0 wt %
   - Varnish (trade name: Saful, manufactured by T&K TOKA) 80.5 wt %
   - Methanol 15.0 wt %
   - Ultraviolet absorbent 2.5 wt %
(trade name: Tinuvin 400, manufactured by Ciba-Geigy Corp.)

The indicator composition was coated on the surface of a sheet of a high-density polyethylene nonwoven fabric by gravure method of using a gravure roll to a coating amount of 10 g/m² after drying, forming an indicator area. The indicator area was dark brown in color.

### (Examples 2 to 10)

Each composition for forming an indicator area were prepared in a similar manner to Example 1, except that hematoxylin used in the plasma-sterilization indicator composition 1 was replaced with the adsorption indicator or metal chelate-titration indicator shown in the following Table 1. The colors of the indicator areas are also shown in the following Table 1.

**Table 1**

| | Component (A) | Display performance | | Discoloration speed | Display performance of sterilization packaging material | Sterilization performance of sterilization packaging material |
|---|---|---|---|---|---|---|
| | | Color before sterilization treatment | Color after sterilization treatment | | Color after sterilization treatment | Growth of microbe |
| Example 1 | Hematoxylin | Dark brown | Yellow | ○ | Yellow | None |
| Example 2 | Mordant Blue 29 | Blue | Purple | ○ | Purple | None |
| Example 3 | Eriochrome Black T | Gray | Yellow | ○ | Yellow | None |
| Example 4 | Xylenol orange | Bluish purple | Red purple | ○ | Red purple | None |
| Example 5 | PAN | Orange | Brown | ○ | Brown | None |
| Example 6 | Congo red | Pink | light pink | ○ | light pink | None |
| Example 7 | Phenol red | Yellowish red | Red | ○ | Red | None |
| Example 8 | Bromocresol purple | Orange | Yellow | ○ | Yellow | None |
| Example 9 | Bromophenol blue | Brown | Yellowish blue | ○ | Yellowish blue | None |
| Example 10 | Methyl red | Reddish brown | Carnation | ○ | Carnation | None |

### (Evaluation of plasma-sterilization indicator)

### 1. Display performance

The nonwoven fabric sheet above was placed in a low-temperature plasma sterilization system [STERRAD-100 (trade name), manufactured by Johnson & Johnson K.K. Medical Company] and sterilized for 75 minutes, and then, the color change in the indicator area was observed.

### 2. Discoloration speed

Immediately after formation of the indicator area, the non-woven fabric sheet having was placed in a low-temperature plasma-sterilization system [STERRAD-100 (trade name), manufactured by Johnson & Johnson K.K. Medical Company] and treated with hydrogen peroxide for 1 minute, and the color change in the indicator area was studied by visual observation. Complete discoloration was designated as ○, while no detection of discoloration by visual observation as ×.

### 3. Sterilization and display performance of sterilization packaging material

A sterilization packaging bag was prepared by using the nonwoven fabric sheet above in one side and a laminate transparent polyester/low-density polyethylene film in the other and heat-sealing them in three directions. A biological indicator (BI) was placed and sealed in the sterilization bag, and the bag was sterilized in a low-temperature plasma sterilization system [STERRAD-100 (trade name), manufactured by Johnson & Johnson K.K. Medical Company] for 75 minutes. The color change in the indicator area after sterilization was observed. The results are summarized in the following Table 2.

Then, the biological indicator was taken out aseptically, placed on a sterilized TSB medium, and incubated at 35°C for 7 days; and presence of the growth of microbe was observed by visual observation. The results are summarized also in Table 1 above. As apparent from Table 1, the plasma-sterilization indicator according to the invention discolors rapidly by plasma sterilization treatment and the discoloration speed is high, indicating that it has a superior display performance. In addition, when the discolored indicator area after evaluation of display performance was preserved in an alkaline environment for 75 minutes, no color change was observed on the indicator in any of the Examples 1 to 10, confirming that the discoloration was irreversible and not dependent on pH.

It was also found that the display function was not disturbed and the efficiency of the plasma sterilization treatment was not affected even when the indicator area was formed as a part of sterilization packaging material.

### (Examples 11 to 20)

Nonwoven fabric sheets and sterilization packaging materials of Examples 11 to 20 were prepared in a similar manner to Example 1, except that the organic metal compound, diisopropoxybis(acetylacetonato)titanium, in the sterilization indicator compositions used in Examples 1 to 10 was replaced with aluminum ethyl acetoacetate diisopropylate, and evaluated in a similar manner to Example 1.

The evaluation results, which were similar to those in Examples 1 to 10, confirmed that it was possible to obtain a color almost the same and to give a nonwoven fabric sheet or a sterilization packaging material favorable in display and sterilization performances even when the organic metal compound was replaced.

### (Example 21)

A nonwoven fabric sheet and a sterilization packaging material were prepared in a similar manner to Example 1, except that the solvent, methanol, in the composition for the indicator area in Example 1 was replaced with ethanol, and evaluated in a similar manner to Example 1.

Although the color before discoloration was gray, the color after sterilization was similar to the color in Example 1, confirming that it was possible to obtain a color and display/sterilization performances similar to those in Example 1 even when the solvent is changed.

### (Example 22)

The following respective components for plasma-sterilization indicator were blended until homogeneity, to give a composition for forming an indicator area.

### (Plasma-sterilization indicator composition 2)

- Hematoxylin [component (A)] 1.2 wt %
- Diisopropoxybis(acetylacetonato)titanium [component (B)] 2.0 wt %
- Polyethylene glycol (PEG200) [component (C)] 3.6 wt %
- Varnish (trade name: NT-VESTA varnish) 72.5 wt %
- Ethanol 18.0 wt %
- Ultraviolet absorbent 3.0 wt %
   (trade name: Tinuvin 400, manufactured by Ciba-Geigy Corp.)

The indicator composition was coated on the surface of a sheet of a high-density polyethylene nonwoven fabric by gravure method of using a gravure roll to a coating amount of 10 g/m² after drying, forming an indicator area. The indicator area was gray in color.

### (Evaluation of plasma-sterilization indicator)

### 1. Display performance

The nonwoven fabric sheet was placed in a low-temperature plasma sterilization system [STERRAD-1005 (trade name), manufactured by Johnson & Johnson K.K. Medical Company] and sterilized for 55 minutes (short cycle), and the color change in the indicator area was observed, confirming that the nonwoven fabric sheet discolored from gray to yellow. The sterilization system is a system designed to give a sterilization performance more uniform on the article to be sterilized.

### 2. Discoloration speed

Immediately after formation of the indicator area, the nonwoven fabric sheet was placed in a low-temperature plasma-sterilization system [STERRAD-100S (trade name), manufactured by Johnson & Johnson K.K. Medical Company] and treated with hydrogen peroxide for 3 minute, and the color change in the indicator area was studied by visual observation, which confirmed that the indicator area in Example 1 discolored completely from gray to yellow.

### 3. Sterilization and display performances of sterilization packaging material

A sterilization packaging bag was prepared by using the nonwoven fabric sheet above in one side and a transparent polyester/low-density polyethylene laminate film in the other and heat-sealing them in three directions. A biological indicator (BI) is placed and sealed in the sterilization bag, and the bag was sterilized in a low-temperature plasma sterilization system [STERRAD-100 (trade name), manufactured by Johnson & Johnson K.K. Medical Company] for 55 minutes. The color change in the indicator area after sterilization was observed. As a result, the indicator area discolored from gray to yellow.

Separately, the biological indicator was taken out aseptically, placed on a sterilized TSB medium, and incubated at 35°C for 7 days; and presence of the growth of microbe was observed by visual observation. As a result, no growth of microbe was observed, confirming that the plasma sterilization treatment was sufficiently effective.

For evaluation of discoloration stability, a rubber glove, assuming the article to be sterilized, was sealed in the sterilization packaging material and subjected to plasma sterilization under a similar condition; the treatment was stopped after 6 minutes from injection of hydrogen peroxide; and the color change in the indicator area was observed by visual observation. As a result, it was confirmed that the area of the indicator area in contact with the rubber glove discolored to yellow similarly to the range not in contact therewith.

The result indicates that the plasma-sterilization indicator according to the invention discolors rapidly by the plasma sterilization treatment and the discoloration speed is high, indicating that it has a superior display performance. In addition, when the discolored indicator area after evaluation of display performance was preserved in an alkaline environment for 55 minutes, no color change of the indicator was observed, confirming that the discoloration was irreversible and not dependent on pH.

It was also found that the display function was not disturbed and the plasma sterilization treatment effect was not affected even when the indicator area is formed as a part of the sterilization packaging material.

### (Comparative Example 1)

A plasma-sterilization indicator composition 2 was prepared in a similar manner to Example 22, except that polyethylene glycol in an amount of 3.6 wt % in the plasma-sterilization indicator composition 2 above was not added and the amount of the varnish added was increased in the same amount. A nonwoven fabric sheet and a sterilization packaging material were obtained by using the composition and evaluated in a similar manner to Example 22, confirmed that:
1. The indicator area discolored from gray to yellow, when the display performance thereof was observed;
2. The indicator area was still gray in color when the treatment was stopped in 3 minutes from injection of hydrogen peroxide and completely discolored to yellow in 6 minutes of treatment, when the color change in the indicator area was observed visually for evaluation of the discoloration speed. As apparent from the results, the indicator of Example 22 is particularly superior in discoloration speed; and
3. Although there were no problems in the color change in the indicator area after sterilization and plasma sterilization treatment effect in the sterilization and display performance tests of the sterilization packaging material, the area in contact with the rubber glove in the indicator area still remained gray in color in the discoloration stability test. The rubber, raw material for the rubber gloves, is known to adsorb hydrogen peroxide and thus is a factor slowing the discoloration, but the indicator according to the invention was less vulnerable to the effects by the material for the article to be sterilized and gave a superior discoloration speed.

### (Example 23)

A nonwoven fabric sheet and a sterilization packaging material were prepared in a similar manner to Example 22, except that the organic metal compound in the sterilization indicator composition 2 obtained in Example 22 above, diisopropoxybis(acetylacetonato)titanium, was replaced with aluminum ethyl acetoacetate diisopropylate, and evaluated in a similar manner to Example 22.

Although the color changed from purple to yellow because the organic metal compound was replaced, a similar effect was observed except color, and the evaluation results confirmed that it was possible to obtain favorable display performance, discoloration speed, sterilization performance, and discoloration stability.

### (Comparative Example 2)

A plasma-sterilization indicator composition was prepared in a similar manner to Example 23, except that the polyethylene glycol used in an amount of 3.6 wt % in the Example 23 was not added and the amount of the varnish added was increased in the same amount; and a nonwoven fabric sheet and a sterilization packaging material were prepared by using the same. Evaluation thereof in a similar manner to Example 22 gave a result that there was no problem in the tests of the display performance and the sterilization and display performance of the sterilization packaging material, but that the indicator area remained purple in color when the treatment was stopped in 3 minutes after hydrogen peroxide injection and turned completely to yellow in color in 6 minutes after treatment in the test for evaluation of the discoloration speed.

In addition, in the discoloration stability test, the area of the indicator area in contact with the rubber glove remained purple in color.

These results confirmed that the plasma-sterilization indicator according to the invention discolored rapidly by plasma sterilization treatment and had a higher discoloration speed and a superior display performance. In addition, the discoloration speed and stability of the plasma-sterilization indicator in the second embodiment including a component (C) were found to be particularly superior.

It was also found that the display function was not disturbed and the plasma sterilization treatment effect was not affected even when the indicator is formed as a part of a sterilization packaging material.

### Industrial applicability

The plasma-sterilization indicator according to the present invention allows definite judgment of whether the article placed in a sterilization packaging material was subjected to plasma sterilization treatment because the discoloration is irreversible, and thus, is useful as a plasma-sterilization indicator because the indicator discoloration speed for display is higher. In addition, the sterilization packaging material according to the present invention, which has an indicator area of the plasma-sterilization indicator formed, allows definite judgment of whether the article in a sterilization packaging material was previously subjected to plasma sterilization treatment because the discoloration in the indicator area is irreversible and has a higher indicator discoloration speed; and thus, is useful as a packaging material for articles such as medical devices, medicines, and foods to be sterilized in various plasma sterilization treatments because the indicator area does not affect the sterilization treatment and has an advantage of allowing definite judgment of whether a plasma sterilization treatment was previously performed.

## Claims

1. A sterilization packaging material capable of containing an article to be sterilized by plasma-sterilization treatment, wherein at least part thereof is made of a gas permeable paper or nonwoven fabric,
and an indicator area of a hydrogen peroxide plasma-sterilization indicator comprising one or more compounds (A) selected from the group consisting of absorption indicators and metal chelate tritration indicators wherein the one or more compounds (A) is selected from hematoxylin, Mordant Blue 29, Eriochrome Black T, xylenol orange, and 1-(2-pyridylazo)-2-naphthol (PAN),
and an organic metal compound (B) which is one or more compound selected from aluminium chelate compounds, titanium chelate compounds, and zirconium chelate compounds is formed thereon.

2. A sterilization packaging material according to claim 1, wherein the indicator area further comprises polyvalent alcohol (C).

3. The hydrogen-peroxide plasma-sterilization indicator according to claim 2, wherein the polyvalent alcohol (C) includes one or more glycols selected from ethylene glycol, diethylene glycol, polyethylene glycol, propylene glycol, and dipropylene glycol.

## Patentansprüche

1. Sterilisationsverpackungsmaterial, das einen durch Plasmasterilisationsbehandlung zu sterilisierenden Artikel aufnehmen kann, wobei zumindest ein Teil davon aus einem gasdurchlässigen Papier oder Vliesstoff besteht, mit einer Indikatorfläche eines Wasserstoffperoxid-Plasmasterilisationsindikators, der eine oder mehrere Verbindungen (A) umfasst, die aus der Gruppe bestehend aus Absorptionsindikatoren und Metallchelattitrationsindikatoren ausgewählt sind, wobei die eine oder mehreren Verbindungen (A) aus Hämatoxylin, Blaubeize 29, Eriochromschwarz T, Xylenolorange und 1-(2-Pyridylazo)-2-naphthol (PAN) ausgewählt sind, auf der eine organische Metallverbindung (B), die aus Aluminiumchelatverbindungen, Titanchelatverbindungen und Zirconiumchelatverbindungen ausgewählt ist, ausgebildet ist.

2. Sterilisationsverpackungsmaterial nach Anspruch 1, bei dem die Indikatorfläche weiterhin mehrwertigen Alkohol (C) umfasst.

3. Wasserstoffperoxid-Plasmasterilisationsindikator nach Anspruch 2, bei dem der mehrwertige Alkohol (C) ein oder mehrere aus Ethylenglycol, Diethylenglycol, Polyethylenglycol, Propylenglycol und Dipropylenglycol ausgewählte Glycole einschließt.

## Revendications

1. Matériau d'emballage de stérilisation capable de contenir un article à stériliser par un traitement de stérilisation de plasma, dans lequel au moins une partie de celui-ci est constitué d'un papier perméable au gaz ou d'un tissu non tissé et une zone d'indication de la stérilisation du plasma de peroxyde d'hydrogène comprenant un ou plusieurs composés (A) choisis parmi le groupe constitué des indicateurs d'absorption et des indicateurs de tritration de chélate métallique dans lequel un ou plusieurs composés (A) est choisi parmi l'hématoxyline, "Mordant Blue 29", "Eriochrome Black T", le xylenol orange et le 1-(2-pyridylazo)-2-naphthol (PAN), et un composé organique métallique (B) qui est un ou plusieurs composés choisis parmi les composés chélate d'aluminium, les composés chélate de titanium et les composés chélate de zirconium.

2. Matériau d'emballage de stérilisation selon la revendication 1, dans lequel la zone d'indication comprend de plus un alcool polyvalent (C).

3. Indicateur de stérilisation de plasma de peroxyde d'hydrogène selon la revendication 2, dans lequel l'alcool polyvalent (C) inclut un ou plusieurs glycols choisi parmi l'éthylène glycol, le diéthylène glycol, le polyéthylène glycol, le propylène glycol et le dipropylène glycol.
